# EUROPÄISCHE PATENTSCHRIFT

(11) **EP 0 812 821 B1**
(45) Veröffentlichungstag und Bekanntmachung des Hinweises auf die Patenterteilung: **29.11.2000**
(21) Anmeldenummer: 97109372.9
(22) Anmeldetag: 10.06.1997
(51) Int. Cl.: C07C 213/04, C07C 219/16

(54) **Kohlensäureesterbetaine und deren Herstellung und Verwendung**
Carbonic ester of betaine, their preparation and use
Ester carbonique de betaine, leur préparation et utilisation

(30) Priorität: 12.06.1996 DE 19623324
(43) Veröffentlichungstag der Anmeldung: 17.12.1997
(73) Patentinhaber: Akzo Nobel N.V., 6824 BM Arnhem (NL)
(72) Erfinder: Ihrig, Klaus, Dr., 69429 Waldbrunn-Schollbrunn (DE); Bergfeld, Manfred, Dr., 63906 Erlenbach-Mechenhard (DE)
(74) Vertreter: Fett, Günter

(56) Entgegenhaltungen:
- EP-A- 0 012 215
- EP-A- 0 074 072
- EP-A- 0 400 435
- GB-A- 379 260

## Beschreibung

Die Erfindung betrifft Kohlensäureesterbetaine und deren Herstellung und Verwendung, vor allem zur Herstellung von Cholinsalzen, insbesondere von Cholinsalzen, die praktisch frei von Nebenprodukten sind.

Cholin, mit welcher Bezeichnung man vielfach die Verbindung 2-Hydroxyethyl-trimethyl-ammoniumhydroxid benennt (s. Römpp "Chemie Lexikon" 9. Auflage, Georg Thieme Verlag Stuttgart, Band 1 (1989), Seite 719 oder Kirk-Othmer "Encyclopedia of Chemical Technology" 3. Ausgabe, Band 6, Seite 19), sowie Salze von Cholin sind bereits seit langem bekannte Verbindungen, die auf den verschiedensten Gebieten eingesetzt werden können.

Bisweilen wird mit Cholin auch das Kation [2-Hydroxy-ethyl]trimethyl-ammonium bezeichnet (s. Beilstein E IV 4, Seite 1443).

Schon die französische Patentschrift 736 107 erwähnt die Verwendung von Cholinsalzen in pharmazeutischen und kosmetischen Cremes, sowie als Zusatzmittel bei Färbe- oder Ausrüstungsverfahren in der Leder- und Textilindustrie.

In der deutschen Offenlegungsschrift 28 49 065 wird die Verwendung von quartären Choliniumsalzen als Leitsalze bei der Elektrolyse gelehrt.

In der DE-A1 3 135 671 wird ein Verfahren zur kontinuierlichen Herstellung von Salzen des Cholins beschrieben, bei dem Trimethylamin, Ethylenoxid und eine Säure in Gegenwart von Wasser bei einem pH-Wert von oberhalb 9 bis 12 und einer Temperatur von 50 bis 80 °C umgesetzt werden. Die so erhaltenen Salze können als Ausgangsstoffe für Pharmazeutika und Futtermittel verwendet werden.

EP-A- 0 400 435 offenbart betainartige Kohlensäurehalbester der Formel 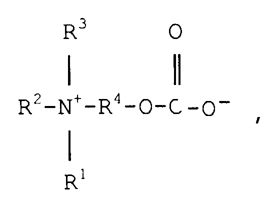 wobei R¹ und R² gleich oder verschieden sind und u.a. für eine C₁- bis C₂₅- Alkylgruppe stehen, R³ u.a. eine C₁- bis C₁₀-Alkylgruppe und R⁴ u.a. eine C₁- bis C₂₅-Alkylengruppe ist, die aus Alkanolaminen und Kohlensäurediestern mit einer Ausbeute von 40 bis 42 % hergestellt werden.

Bei der Herstellung von Cholin geht man im allgemeinen so vor, daß man Trimethylamin in Gegenwart von Wasser mit Ethylenoxid umsetzt. Führt man die Umsetzung von Trimethylamin und Ethylenoxid in Gegenwart von Kohlenstoffdioxid und Wasser durch, erhält man direkt Cholinbicarbonat bzw. Cholincarbonat, also Cholinsalze der Kohlensäure. Durch Zusatz von Säuren wie Salzsäure oder organischen Säuren wie z.B. Weinsäure erhält man dann die entsprechenden Cholinsalze.

Von Nachteil bei diesen Verfahren ist, daß sich dabei eine Reihe von Nebenprodukten bildet, z.B. Oligomere, d.h. Verbindungen, bei denen mehr als 1 Molekül Ethylenoxid angelagert ist. Weitere Nebenprodukte sind Ethylenglykol, Polyethylenglykol, Chlorhydrine u.a.m.

Derartige Nebenprodukte machen die so erhaltenen Cholinsalze für viele Anwendungen ungeeignet. Es sind deshalb z.T. sehr aufwendige Reinigungsschritte erforderlich.

Eines der wichtigsten Cholinsalze, nämlich Cholinchlorid, kann nach den oben geschilderten Verfahren gewonnen, aber auch, wie in der FR-PS 1 458 127 angegeben, durch Umsetzung von Trimethylammoniumchlorid mit Ethylenoxid erhalten werden. Aber auch bei diesem Verfahren gibt es Probleme mit den Nebenprodukten.

Meistens werden diese Verunreinigungen erst bei der Herstellung von Folgeprodukten entfernt, z.B. bei der Überführung von Cholinmono- oder Cholinbicarbonat in Cholinbitartrat. Die Salze werden durch Kristallisation gereinigt, wobei sich die Mutterlauge immer mehr mit Oligomeren und sonstigen Nebenprodukten anreichert, was u.a. den Nachteil mit sich bringt, daß man die Mutterlauge nur bedingt im Kreislauf fahren kann. Man muß entweder die Mutterlauge separat aufarbeiten oder stets einen nicht unbeträchtlichen Teil, wenn man doch im Kreislauf arbeiten will, entnehmen und separat aufarbeiten, um das Nebenproduktniveau konstant zu halten.

Die oben beschriebenen Nachteile treten auch auf, wenn man Ethylenoxid mit Aminen der Formel (II) 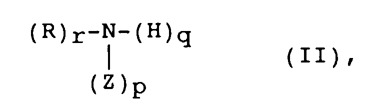 worin R eine Alkylgruppe mit 1 bis 18 C-Atomen und Z eine Hydroxyalkylgruppe mit 1 bis 6 C-Atomen ist, r, p und q ganze Zahlen darstellen, r+p+q=3 ist, r Werte von 0 bis 3, p Werte von 3 bis 0 und q Werte von 0 bis 2 aufweist, umsetzt.

Es besteht deshalb noch ein Bedürfnis nach Verfahren, bei welchen die oben erwähnten Nachteile nicht auftreten, bzw. nach einer Verbindung, welche zur Herstellung von Salzen der Formel (III) 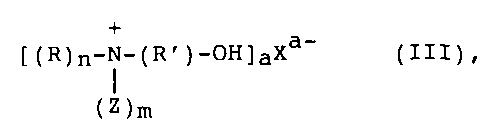 worin R eine oder verschiedene Alkylgruppen mit 1 bis 18 C-Atomen, R' eine Alkylengruppe mit 2 bis 12 C-Atomen und Z eine Hydroxyalkylgruppe mit 1 bis 12 C-Atomen ist, m und n ganze Zahlen darstellen, m Werte von 0 bis 3 und n Werte von 3 bis 0 aufweist, m+n=3 ist, X das Anion einer organischen oder anorganischen Säure darstellt, und a ganze Zahlen von 1 bis 3 bedeutet, eingesetzt werden kann, wobei die anfallenden Salze als im wesentlichen reine Produkte anfallen, die nicht noch aufwendig gereinigt werden müssen. Es besteht ferner ein Bedürfnis auch nach einer solchen Verbindung, die selbst bereits weitgehend frei von Oligomeren oder sonstigen Verunreinigungen ist. Aufgabe der Erfindung ist es somit, ein Verfahren zur Herstellung einer spezifischen Verbindung zur Verfügung zu stellen, die praktisch frei von Oligomeren und sonstigen Nebenprodukten ist und direkt in Salze der Formel (III) übergeführt werden kann. Aufgabe der Erfindung ist es weiterhin, ein Verfahren zur Herstellung von Salzen der Formel (III) zur Verfügung zu stellen, bei dem die Salze bereits direkt ohne entsprechende Verunreinigungen anfallen, so daß die bisher notwendigen Reinigungsverfahren und die Entsorgung von Nebenprodukten bzw. die Aufarbeitung von Mutterlaugen entfällt.

Eine weitere Aufgabe der Erfindung ist es, ein Verfahren zur Herstellung einer solchen Verbindung zur Verfügung zu stellen, das wirtschaftlich arbeitet, bei dem die Ausbeute sehr hoch, d.h. nahezu quantitativ ist und bei dem weniger Aufwand betrieben werden muß, sowohl bei der Aufarbeitung des Produkts selbst als auch bei der Isolierung der daraus hergestellten Folgeprodukte, insbesondere der Salze.

Diese Aufgabe wird gelöst durch ein Verfahren zur Herstellung von Kohlensäureesterbetainen der Formel (I) 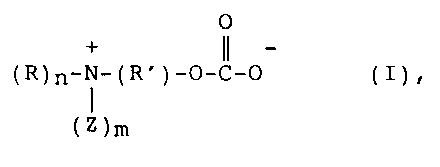 worin R eine oder verschiedene Alkylgruppen mit 1 bis 18 C-Atomen, R' eine Alkylengruppe mit 2 bis 12 C-Atomen und Z eine Hydroxyalkylgruppe mit 1 bis 12 C-Atomen ist, m und n ganze Zahlen darstellen, m Werte von 0 bis 3 und n Werte von 3 bis 0 aufweist, und m+n=3 ist, dadurch gekennzeichnet, daß man ein Amin der Formel (II) 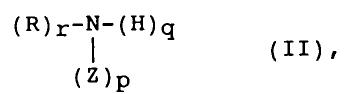 in der R und Z die oben angegebene Bedeutung haben, r, p und q ganze Zahlen darstellen, r+p+q=3 ist, r Werte von 0 bis 3, p Werte von 3 bis 0 und q Werte von 0 bis 2 aufweist, mit einem Alkylenoxid mit 2 bis 12 C-Atomen in Gegenwart von Kohlendioxid in einem im wesentlichen inerten Lösungsmittel und in Abwesenheit von Wasser umsetzt. Als Alkylenoxid wird Ethylenoxid bevorzugt. Es ist vorteilhaft, das Alkylenoxid und Kohlendioxid im Überschuß, bezogen auf das Amin einzusetzen, wobei der Überschuß an Alkylenoxid vorzugsweise 1 - 200 Mol% beträgt. Der Überschuß an Kohlendioxid wird vorzugsweise dadurch gewährleistet, daß man im Reaktionsgefäß einen Überdruck an Kohlendioxid einstellt, der vorzugsweise während der gesamten Reaktion konstant ist.

Die Temperatur, bei welcher die Reaktion durchgeführt werden kann, läßt sich innerhalb weiter Bereiche variieren, z.B. von Zimmertemperatur bis 150 °C. Vorzugsweise wird jedoch die Reaktion bei einer Temperatur von 40 - 80 °C durchgeführt.

Der Druck, welcher vorzugsweise mit Kohlenstoffdioxid eingestellt wird, liegt vorzugsweise in einem Bereich von 1 bis 5 bar.

Die Reaktion wird in Abwesenheit von Wasser durchgeführt, wobei Abwesenheit von Wasser bedeutet, daß im wesentlichen kein Wasser vorhanden ist; es findet also kein Zufügen von Wasser statt. Geringe Restmengen an Wasser im eingesetzten Lösungsmittel oder Feuchtigkeit in den verwendeten Reagenzien sind bis zu einem gewissen Grade unschädlich. Die Menge an Wasser sollte jedoch nicht mehr als 0,1 Mol%, bezogen auf das eingesetzte Amin betragen.

Die Umsetzung wird in Gegenwart von Lösungsmitteln durchgeführt, wobei als Lösungsmittel Methanol besonders geeignet ist. Weitere brauchbare Lösungsmittel sind z.B. n-Propanol, Isopropanol und n-Butanol. Im allgemeinen sind polare, protische und aprotische Lösungsmittel geeignet, die unter Reaktionsbedingungen im wesentlichen inert gegenüber den eingesetzten Chemikalien und dem entsprechenden Produkt sind. Mit anderen Worten: Es sind solche Lösungsmittel geeignet, die unter den Reaktionsbedingungen mit den eingesetzten Chemikalien und dem entsprechenden Produkt möglichst keine oder nur geringe oder sehr geringe Reaktionen eingehen.

Die Verwendung von einem Inertgas im Reaktionsraum ist möglich. So kann u.a. die Reaktionskammer zunächst mit einem Inertgas gespült werden, z.B. Stickstoff oder Argon. Das im Reaktionsgefäß nach dem Spülprozeß vorhandene Inertgas kann während der Reaktion darin verbleiben.

Die Herstellung der Kohlensäureesterbetaine kann z.B. auf folgende Weise geschehen.

Ein geeignetes Reaktionsgefäß wird zunächst mit einem Inertgas, z.B. Stickstoff oder Argon gespült. Sodann wird das Lösungsmittel vorgelegt, insbesondere Methanol, und das Amin entweder gasförmig oder flüssig in das Lösungsmittel eindosiert. Sodann wird auf das System Kohlendioxid gasförmig aufgedrückt. Zweckmäßigerweise ist das Reaktionsgefäß ein Autoklav, so daß Drucke z.B. von 2 bar und mehr eingestellt werden können. Sodann wird eine Temperatur von beispielsweise 40 bis 80 °C eingestellt und unter Rühren Alkylenoxid zudosiert, das in flüssiger oder auch in gasförmiger Form eingebracht werden kann. Während der Zuleitung des Alkylenoxids wird die Temperatur kontrolliert, wobei man durch Abführung der Reaktionswärme die Reaktionstemperatur vorzugsweise konstant hält.

Nach vollständiger Umsetzung des eingesetzten Amins wird entspannt und unter Unterdruck mit Inertgas gestrippt, wobei überschüssiges Alkylenoxid und Kohlendioxid entfernt werden. Man enthält eine klare farblose Lösung des Produkts, in der sich die Reinheit und der Gehalt an Kohlensäureesterbetain durch Flüssigkeitschromatographie eindeutig bestimmen lassen. Nach Abdestillieren des Lösungsmittels, was vorzugsweise unter Unterdruck geschieht, erhält man das Betain, das zunächst in Form eines zähen farblosen Öls anfällt, das nach einer bestimmten Zeit zu einem farblosen Feststoff erstarrt. Beim Einsatz von längerkettigen Epoxiden können auch flüssige Betaine entstehen.

Die Reaktion kann auch kontinuierlich, d.h. insbesondere vollkontinuierlich durchgeführt werden.

Durch das erfindungsgemäße Verfahren werden erstmalig Kohlensäureesterbetaine der Formel (I) 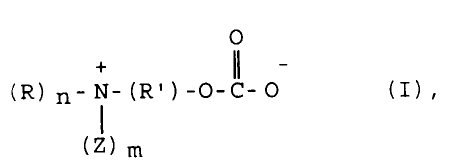 hergestellt, worin R, R', Z, m und n die vorstehend genannte Bedeutung haben, mit Ausnahme der Verbindungen für welche n = 3 ist.

Durch Einwirkung von Säuren mit pKₛ <6,46 auf die Esterbetaine der Formel (I) lassen sich die Salze der Formel (III) herstellen.

### Verwendung der Salze der Formel (III) am Beispiel von Cholinbitartrat:

Wie die meisten der bereits genannten Cholinsalze z. B. Cholinchlorid, so findet auch Cholinbitartrat (s. Formel IV) hauptsächlich Verwendung als Zusatz in Tierfuttern, insbesondere in Katzen- und Hühnerfuttern. Daneben wird Cholinbitartrat aber auch für pharmazeutische Zwecke zur Therapie von Patienten mit Leberzirrhose oder anderen Lebererkrankungen verwendet. Eine größere Menge an Cholinbitartrat geht außerdem in Softdrinks und in sog. Powerdrinks.

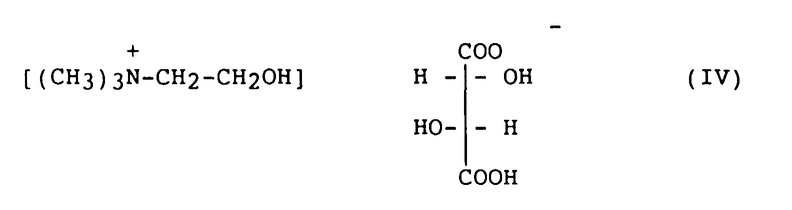

Es war besonders überraschend, daß gemäß der Erfindung ein Produkt in Gestalt eines Kohlensäuremonocholinesterbetains zugänglich ist, das selbst bereits einen sehr hohen Reinheitsgrad aufweist, d.h. praktisch keine Oligomere und sonstige Nebenprodukte enthält. Der Reinheitsgrad dieser Substanz macht sich besonders vorteilhaft bemerkbar, wenn man aus dem Kohlensäuremonocholinesterbetain in an sich bekannter Weise Abkömmlinge des Cholins herstellen möchte, wie z.B. Salze, insbesondere das bereits erwähnte Cholinbitartrat oder Cholincitrat.

Deshalb ist ein weiterer Gegenstand der Erfindung die Verwendung des Kohlensäuremonocholinesterbetains zur Herstellung von Salzen der Formel (III), z.B. zur Herstellung von Cholinsalzen.

Bei der Herstellung von beispielsweise Cholinbitartrat mußte man bisher bei der Kristallisation stets die Mutterlauge in besonderen Schritten reinigen oder sogar verwerfen, wenn sich die Reinigung nicht lohnte, da sich in der Mutterlauge Oligomere und sonstige Nebenprodukte anreicherten. Aufgrund der Erfindung ist es möglich, Cholinbitartrat kristallin zu gewinnen und die dabei anfallende Mutterlauge im Kreislauf zu fahren, ohne daß Oligomere oder sonstige Nebenprodukte abgetrennt werden müßten. Dies bringt erhebliche Vorteile bei der Verfahrensdurchführung, insbesondere höhere Ausbeuten, reinere Produkte, Energieeinsparung usw.

Das erfindungsgemäße Verfahren läuft erheblich schneller ab, als die bekannten Verfahren zur Herstellung beispielsweise von Cholin oder von Cholincarbonaten.

Zur Erläuterung der Erfindung dienen folgende Beispiele:

### Herstellung von Cholinbitartrat:

1. Klassische Route
   Zunächst erfolgt die Umsetzung von Trimethylamin und Ethylenoxid in wasserfreiem Methanol zu (CH₃)₃N⁺CH₂CH₂O⁻. Diese Lösung wird anschließend durch Zugabe einer stöchiometrischen Menge an Weinsäure (gelöst in Methanol) in Cholinbitartrat überführt, welches aus der methanolischen Lösung auskristallisiert.
2. Route über das erfindungsgemäße Kohlensäuremonocholinesterbetain
   Die Lösung des Kohlensäuremonocholinesterbetains wird bei ca. 40 °C mit einer Lösung von Weinsäure in Methanol versetzt wobei es zu einer starken CO₂-Entwicklung kommt. Gleichzeitig kühlt sich die Lösung ab und nach ca. 50 % Zugabe der Gesamtmenge an Weinsäure beginnt das Cholinbitartrat zu kristallisieren. Nach beendeter Zugabe von Weinsäure wird noch ca. 1 Std. bei Raumtemperatur gerührt und dann filtriert. Die Mutterlauge wird auf ca. 50 eingedampft und als Lösungsmittel für die Weinsäure verwendet, die im nächsten Versuch eingesetzt wird. Die Ausbeuten können durch diese Verfahrensweise von ca. 93 ohne Mutterlaugenrecycling auf >99 % mit Mutterlaugenrecycling gesteigert werden.

### Ausführungsbeispiele:

### Beispiel 1: Kohlensäuremonocholinesterbetain

In einem 1 1 Stahlautoklaven, der zuvor mit Stickstoff inertisiert wurde, löste man 62,3 g (1,054 mol) gasförmiges Trimethylamin in 246 g technischem Methanol (Wassergehalt <0,05 %). Diese Lösung wurde anschließend unter Rühren auf Reaktionstemperatur (43-48 °C) gebracht und mit 2 bar Kohlendioxid beaufschlagt; dieser Druck wurde über die gesamte Reaktionszeit konstant gehalten. Danach wurden über einen Zeitraum von 30 min 51,2 g (1,162 mol) flüssiges Ethylenoxid zudosiert. Nach beendeter Zudosierung wurde noch 45 min bei ca. 45 °C nachreagiert. Insgesamt wurden 48,3 g (1,096 mol) Kohlendioxid aufgenommen. Anschließend wurde die Mischung zum Entfernen von überschüsssigem Trimethylamin und Kohlendioxid für 1 Stunde im Vakuum bei 30 °C mit Stickstoff gestrippt. Durch quantitative Flüssigkeitschromatographie wurden der Gehalt und die Reinheit des erhaltenen Kohlensäuremonocholinesterbetains bestimmt. Bei praktisch quantitativer Ausbeute betrug die Reinheit >99 %; es konnten keine höheren Ethoxylate und keine Reaktionsprodukte zwischen dem Lösungsmittel und Ethylenoxid (z.B. Ethylenglykolmonomethylether) nachgewiesen werden.

In einem zweiten Reaktionsgefäß wurden 158,4 g (1 eq.) Weinsäure in 210 g Methanol bei 40 °C gelöst. Diese Lösung wurde sodann langsam in die 30 °C warme Reaktionslösung des Kohlensäuremonocholinesterbetains eindosiert, wodurch eine kräftige CO₂-Entwicklung und eine Abkühlung der Reaktionslösung eintrat. Nach ca. 50 % Zugabe der Gesamtmenge an Weinsäure setzte die Kristallisation von Cholinbitartrat ein. Dieses wurde abfiltriert und getrocknet. Ausbeute: 245,3 g (91,9 % d.Th.) Cholinbitartrat mit Schmelzbereich 146,5-147,5 °C.

### Beispiel 2:

Die in Beispiel 1 angefallene Mutterlauge (339,5 g) wurde bis auf ein Gesamtgewicht von 210 g eingedampft und als Lösungsmittel für 158,4 g Weinsäure verwendet. Diese Lösung wurde dann zur Herstellung von Cholinbitartrat ausgehend von Kohlensäuremonocholinesterbetain verwendet, das zuvor in einem, wie in Beispiel 1 beschriebenen, Ansatz hergestellt wurde. In diesem Fall konnten 266.8 g (99,6 %) Cholinbitartrat erhalten werden.

### Vergleichsbeispiel unter Standardbedingungen ohne CO₂-Beaufschlagung:

In einem 1 l Stahlautoklaven, der zuvor mit Stickstoff inertisiert wurde, löste man 63,09 g (1,067 mol) gasförmiges Trimethylamin in 246 g technischem Methanol. Diese Lösung wurde anschließend unter Rühren auf Reaktionstemperatur (43-48 °C) gebracht und mit 2 bar Stickstoff beaufschlagt. Danach wurden über einen Zeitraum von 30 min 51,6 g (1,171 mol) flüssiges Ethylenoxid zudosiert. Nach beendeter Zudosierung wurde noch 45 min bei ca. 45 °C nachreagiert. Anschließend wurde die Mischung zum Entfernen von überschüsssigem Trimethylamin für 1 Stunde im Vakuum bei 30 °C mit Stickstoff gestrippt. Durch quantitative Flüssigkeitschromatographie konnte gezeigt werden, daß die Reaktionslösung etwa 86% (CH₃)₃N⁺CH₂CH₂O⁻, ca. 4,4 % (CH₃)₃N⁺CH₂CH₂O-CH₂CH₂O⁻ und ca. 2 % 2-Methoxyethanol enthielt. Die restlichen 7,6 % setzten sich aus einer Reihe von Produkten zusammmen, die nicht näher identifiziert wurden. In einem zweiten Reaktionsgefäß wurden 159,3 g (1 eq.) Weinsäure in 214 g Methanol bei 40 °C gelöst. Diese Lösung wurde sodann langsam in die 30 °C warme Reaktionslösung eindosiert. Das gebildete Cholinbitartrat wurde abfiltriert und getrocknet. Ausbeute: 221,1 g (81,8 % d.Th.) Cholinbitartrat mit Schmelzbereich 146,5 - 147,2 °C.

### Verwendung anderer Alkohole als Lösungsmittel

Neben den bereits oben näher beschriebenen Versuchen mit Methanol als Lösungsmittel wurden zusätzlich noch einige Versuche mit Isopropanol, n-Propanol und n-Butanol in Gegenwart von CO₂ und Abwesenheit von Wasser durchgeführt.

Die Reaktionsbedingungen waren analog zu denen, die bereits in Beispiel 1 beschriebenen wurden. Ein Recycling der Mutterlaugen wurde bei diesen Ansätzen jedoch nicht durchgeführt. In allen Fällen war die Reinheit des erhaltenen Kohlensäuremonocholinesterbetains laut präparativer Flüssigkeitschromatographie >99 %. Im Gegensatz zu den Versuchen mit Methanol als Lösungsmittel, setzte in diesen Fällen die Kristallisation von Cholinbitartrat bereits nach Zugabe von ca. 20 der benötigten Weinsäuremenge ein. Die Ausbeuten an Cholinbitartrat in Abhängigkeit vom verwendeten Lösungsmittel waren dabei wie folgt.

| | | |
|---|---|---|
| Methanol | 91,9 %; | Fp.: 146,5-147,5 °C |
| Isopropanol | 89,7 %; | Fp.: 147,2-147,9 °C |
| n-Propanol | 93,3 %; | Fp.: 147,3-148,2 °C |
| n-Butanol | 95,4 %; | Fp.: 147,9-148,3 °C |

In analoger Weise lassen sich durch Zugabe entsprechender Mengen an Säure u. a. folgende Cholinsalze herstellen: Tricholincitrat, Bis-cholintartrat, Bis-cholinhydrogenphosphat, Cholindihydrogenphosphat, Cholindihydrogencitrat, Bis-cholinhydrogencitrat, Cholingluconat, Cholinsalicylat, Cholincarboxymethylcellulose und Cholinascorbat.

### Beispiel 3:

### N,N-Dimethyl-N-(2-hydroxyethyl)-N-(1-yl-ethan-2-monocarbonat)-ammonium (A)

In einem 1 1 Büchi-Stahlautoklaven mit Innenthermometer, Gasturbine, Amin-Zuleitung, Ethylenoxid (EO)-Zuleitung und CO₂-Zuleitung, der zuvor mit Stickstoff inertisiert wurde, wurden zunächst 550 ml Isopropanol (Wassergehalt <0,1 %) vorgelegt. Nach Verschließen des Reaktors wurde mittels einer Membranpumpe flüssiges Dimethylamin (29,30 g = 0,65 mol) in den Reaktor eindosiert. Anschließend wurde der Reaktor auf ca. 50 °C erwärmt und unter Rühren wurde ein Druck von 2 bar (absolut) CO₂ eingestellt. Bei geöffnetem CO₂-Ventil wurden dann innerhalb von ca. 30 min mittels einer weiteren Membranpumpe 57,28 g (1,3 mol) Ethylenoxid eindosiert. Der CO₂-Verbrauch während der Reaktion wurde mit Hilfe eines Durchflußreglers gemessen und geregelt. Nach ca. 1,5 Std. Gesamtreaktionszeit war die theoretische Menge an CO₂ (28,6 g entspricht 0,65 mol) von der Reaktionslösung aufgenommen und es konnte kein zusätzlicher Verbrauch mehr festgestellt werden. Nach insgesamt 2 Std. Reaktionszeit wurde der Reaktor abgekühlt, die Lösung aus dem Reaktor entnommen und am Rotationsverdampfer vollständig eingedampft. Als Rückstand erhielt man ein gelbliches zähes Öl, das nach längerem Stehen allmählich zu kristallisieren begann. Die durch GPC- und NMR-Analyse bestimmte Ausbeute an Produkt (A) betrug 94 %.

### Beispiel 4:

### N-Methyl-N,N-bis(2-hydroxyethyl)-N-(1-yl-ethan-2-monocarbonat)-ammonium (B)

In einem Versuch, der entsprechend den Bedingungen von Beispiel 3 durchgeführt wurde, setzte man in 550 ml Isopropanol 31,06 g (1,0 mol) Monomethylamin mit 132,18 g (3,0 mol) Ethylenoxid und CO₂ (2 bar absolut) um. Nach 2 Std. (100 % CO₂-Aufnahme) Reaktionszeit und entsprechender Aufarbeitung erhielt man als Rohprodukt 196,8 g (94,8 %) eines gelben zähen Öls. Nach GPC-Analyse und NMR-Spektroskopie wurde das gewünschte Produkt (B) in einer Ausbeute von 90,5 % erhalten.

### Beispiel 5:

### N,N,N-Tris(2-hydroxyethyl)-N-(1-yl-ethan-2-monocarbonat)-ammonium (C)

In dem bereits in Beispiel 1 beschriebenen Reaktor wurden 149,2 g (1,0 mol) Triethanolamin in 550 ml Isopropanol vorgelegt und bei ca. 50 °C unter CO₂-Druck (2 bar absolut) mit 44,06 g (1 mol) Ethylenoxid umgesetzt. Nach einer Reaktionszeit von 2 Std. erhielt man als Rohprodukt 241 g eines gelblichen Öls, das allmählich erstarrte. Die GPC-Analyse und das NMR zeigten, daß das gewünschte Produkt (C) mit einer Selektivität von ca. 96 % und mit einer Ausbeute von 97 gebildet worden war.

### Beispiel 6:

### N,N-Dibutyl-N-(2-hydroxypropyl)-N-(1-yl-ethan-2-monocarbonat)-ammonium (D)

Ein 1000 ml Büchi-Reaktor wurde mit Stickstoff gespült und dann mit 400 ml Methanol und 37,46 g (0,2 mol) N,N-Dibutyl-N-(2-hydroxypropyl)amin (DBHPA) gefüllt. Nach Verschließen des Reaktors wurde die DBHPA-Lösung auf 55 °C aufgeheizt und innerhalb von 10 min wurden 19,44 g (0,4 mol) Ethylenoxid mittels einer Membranpumpe eindosiert. Anschließend wurde mit CO₂ ein Gesamtdruck von ca. 2 bar eingestellt. Bei geöffnetem CO₂-Ventil ließ man die Reaktion ablaufen. Nach einer Gesamtreaktionszeit von ca. 31 Std. wurde die Reaktion beendet, das Lösungsmittel bei 50 °C im Vakuum vollständig abgedampft und das Produkt (D) in einer Ausbeute von 91,2 % erhalten.

### Beispiel 7:

### N,N-Dibutyl-N-(2-hydroxybutyl)-N-(1-yl-ethan-2-monocarbonat)-ammonium (E)

Ein 1000 ml Büchi-Reaktor wurde mit Stickstoff gespült und dann mit 250 ml Methanol und 40,27 g (0,2 mol) N,N-Dibutyl-N-(2-hydroxybutyl)amin (DBHBA) gefüllt. Nach Verschließen des Reaktors wurde die DBHBA-Lösung auf 55 °C aufgeheizt und innerhalb von 10 min wurden 17,6 g (0,4 mol) Ethylenoxid mittels einer Membranpumpe eindosiert. Anschließend wurde mit CO₂ ein Gesamtdruck von 2 bar eingestellt. Bei geöffnetem CO₂-Ventil ließ man die Reaktion über Nacht laufen. Nach einer Gesamtreaktionszeit von ca. 31 Std. wurde die Reaktion beendet und das Lösungsmittel bei 50 °C im Vakuum vollständig abgedampft. Die mit GPC-Analyse bestimmte Ausbeute an Produkt (E) betrug 74,8 %.

### Beispiel 8:

### N,N-Dimethyl-N-lauryl-N-(1-yl-ethan-2-monocarbonat) ammonium (F)

In einem 1000 ml Büchi-Reaktor wurden 106,7 g (0,5 mol) N,N-Dimethyllaurylamin in 550 ml Isopropanol gelöst. Anschließend wurde die Lösung auf 70 °C erwärmt und unter Rühren ein Druck von 2 bar (absolut) Kohlendioxid eingestellt. Bei geöffnetem Kohlendioxid-Ventil wurden dann innerhalb von etwa 20 Minuten mit einer Membranpumpe 24,2 g (0,55 mol) Ethylenoxid zudosiert. Nach einer Gesamtreaktionszeit von etwa 4 Std. waren 22 g (0,5 mol) Kohlendioxid von der Reaktionslösung aufgenommen. Die Ausbeute an Produkt (F) betrug 93 %.

## Patentansprüche

1. Verfahren zur Herstellung von Kohlensäureesterbetainen der Formel (I) 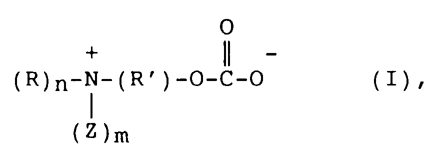 worin R eine oder verschiedene Alkylgruppen mit 1 bis 18 C-Atomen, R' eine Alkylengruppe mit 2 bis 12 C-Atomen und Z eine Hydroxyalkylgruppe mit 1 bis 12 C-Atomen ist, m und n ganze Zahlen darstellen, m Werte von 0 bis 3 und n Werte von 3 bis 0 aufweist, und m+n=3 ist, dadurch gekennzeichnet, daß man ein Amin der Formel (II) 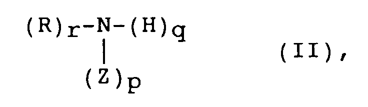 in der R und Z die oben angegebene Bedeutung haben, r, p und q ganze Zahlen darstellen, r+p+q=3 ist, r Werte von 0 bis 3, p Werte von 3 bis 0 und q Werte von 0 bis 2 aufweist, mit einem Alkylenoxid mit 2 bis 12 C-Atomen in Gegenwart von Kohlendioxid in einem im wesentlichen inerten Lösungsmittel und in Abwesenheit von Wasser umsetzt.

2. Verfahren nach Anspruch 1, dadurch gekennzeichnet, daß das Alkylenoxid Ethylenoxid ist.

3. Verfahren nach Anspruch 1 und 2, dadurch gekennzeichnet, daß man die Umsetzung in einem organischen Lösungsmittel vornimmt.

4. Verfahren nach Anspruch 3, dadurch gekennzeichnet, daß man als organisches Lösungsmittel Methanol verwendet.

5. Verfahren nach einem der Ansprüche 1 bis 4, dadurch gekennzeichnet, daß man die Umsetzung mit einem Überschuß an Alkylenoxid und einem Überschuß an Kohlendioxid, bezogen auf das eingesetzte Amin, durchführt.

6. Verfahren nach Anspruch 5, dadurch gekennzeichnet, daß man einen Überschuß von 1 bis 200 Mol% Alkylenoxid und 10 bis 800 Mol% Kohlendioxid verwendet.

7. Verfahren nach einem der Ansprüche 1 bis 6, dadurch gekennzeichnet, daß man die Umsetzung bei einer Temperatur von Raumtemperatur bis 150 °C durchführt.

8. Verfahren nach einem der Ansprüche 1 bis 7, dadurch gekennzeichnet, daß man die Umsetzung bei einer Temperatur von 40 bis 80 °C durchführt.

9. Verfahren nach einem der Ansprüche 1 bis 8, dadurch gekennzeichnet, daß der mit Kohlendioxid eingestellte Druck im Bereich von 1 bis 5 bar liegt.

10. Verfahren nach einem der Ansprüche 1 bis 9, dadurch gekennzeichnet, daß die Reaktionskammer zunächst mit einem Inertgas, vorzugsweise Stickstoff oder Argon, gespült wird, das während der Reaktion in der Kammer verbleiben kann.

11. Verfahren zur Herstellung von Salzen der Formel (III) 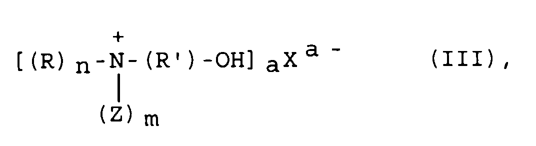 worin R eine oder verschiedene Alkylgruppen mit 1 bis 18 C-Atomen, R' eine Alkylengruppe mit 2 bis 12 C-Atomen und Z eine Hydroxyalkylgruppe mit 1 bis 12 C-Atomen ist, m und n ganze Zahlen darstellen, m Werte von 0 bis 3 und n Werte von 3 bis 0 aufweist, m + n = 3 ist, X das Anion einer organischen oder anorganischen Säure und a ganze Zahlen von 1 bis 3 bedeutet, dadurch gekennzeichnet, daß man zuerst Kohlensäureesterbetaine der Formel (I) 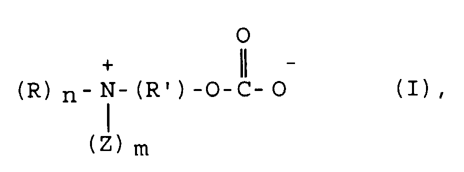 durch Umsetzung eines Amins der Formel (II) 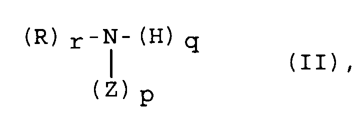 in der R und Z die oben angegebene Bedeutung haben, r, p und q ganze Zahlen darstellen, r + p + q = 3 ist, r Werte von 0 bis 3, p Werte von 3 bis 0 und q Werte von 0 bis 2 aufweist, mit einem Alkylenoxid mit 2 bis 12 C-Atomen in Gegenwart von Kohlendioxid in einem im wesentlichen inerten Lösungsmittel und in Abwesenheit von Wasser herstellt, wonach man Säuren HₐX^{a} mit einem pKₛ <6,46, worin a und X die oben angegebene Bedeutung haben, auf die Kohlensäureesterbetaine der Formel (I) einwirken läßt.

12. Verfahren nach Anspruch 11 zur Herstellung von Cholinbitartrat.

13. Verfahren nach Anspruch 11 zur Herstellung von Tricholincitrat, Bis-cholintartrat, Bis-cholinhydrogenphosphat, Cholindihydrogenphosphat, Bis-cholinhydrogencitrat, Cholindihydrogencitrat, Cholingluconat, Cholinsalicylat, Cholinchlorid und Cholinascorbat.

14. Verfahren nach Anspruch 11 zur Herstellung von Cholincarboxymethylcellulose.

15. Kohlensäureesterbetaine der Formel (I) 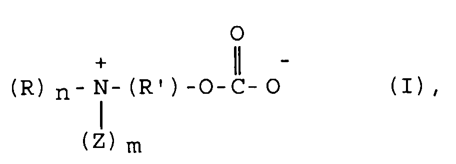 worin R, R', Z, m und n die in Anspruch 1 angegebene Bedeutung haben, mit Ausnahme der Verbindungen für welche n = 3 ist.

## Claims

1. A process for the preparation of carbonic acid ester betaines according to formula (I) 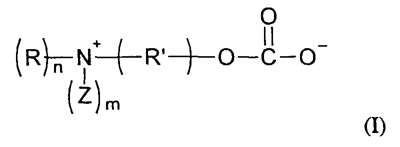 wherein R represents one or more alkyl groups with 1 to 18 C-atoms, R' is an alkylene group with 2 to 12 C-atoms, and Z is a hydroxyalkyl group with 1 to 12 C-atoms, m and n stand for integers, m has a value of 0 to 3 and n has a value of 3 to 0, and m+n=3, characterised in that an amine according to formula (II) 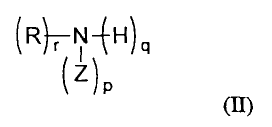 wherein R and Z have the meaning given above, r, p, and q stand for integers, r+p+q=3, r has a value of 0 to 3, p has a value of 3 to 0, and q has a value of 0 to 2, is reacted with an alkylene oxide with 2 to 12 C-atoms in the presence of carbon dioxide in an essentially inert solvent and in the absence of water.

2. A process according to claim 1, characterised in that the alkylene oxide is ethylene oxide.

3. A process according to claims 1 and 2, characterised in that the reaction is carried out in an organic solvent.

4. A process according to claim 3, characterised in that the organic solvent used is methanol.

5. A process according to any one of claims 1 to 4, characterised in that the reaction is carried out with an excess of alkylene oxide and an excess of carbon dioxide, calculated on the amine used.

6. A process according to claim 5, characterised in that an excess of 1 to 200 mole% of alkylene oxide and of 10 to 800 mole% of carbon dioxide is used.

7. A process according to any one of claims 1 to 6, characterised in that the reaction is carried out at a temperature from room temperature to 150°C.

8. A process according to any one of claims 1 to 7, characterised in that the reaction is carried out at a temperature of 40 to 80°C.

9. A process according to any one of claims 1 to 8, characterised in that the pressure set with carbon dioxide is in the range of 1 to 5 bar.

10. A process according to any one of claims 1 to 9, characterised in that the reaction chamber is first of all flushed with an inert gas, preferably nitrogen or argon, which may remain in the chamber during the reaction.

11. A process for the preparation of salts according to formula (III) 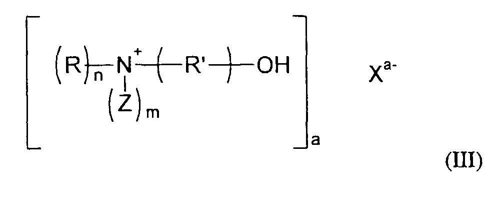 wherein R represents one or more alkyl groups with 1 to 18 C-atoms, R' is an alkylene group with 2 to 12 C-atoms, and Z is an hydroxyalkyl group with 1 to 12 C-atoms, m and n stand for integers, m has a value of 0 to 3 and n has a value of 3 to 0, m + n = 3, X stands for the anion of an organic or inorganic acid, and a has the meaning of integers of 1 to 3, characterised in that first a carbonic acid ester betaine according to formula (I) 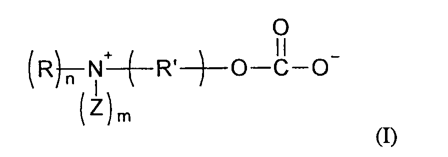 is prepared by the reaction of an amine according to formula (II) 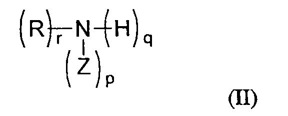 wherein R and Z have the above-indicated meaning, r, p, and q stand for integers, r + p + q = 3, r has a value of 0 to 3, p has a value of 3 to 0, and q has a value of 0 to 2, with an alkylene oxide with 2 to 12 C-atoms in the presence of carbon dioxide in an essentially inert solvent and in the absence of water, after which acids HₐX^{a} having a pKs <6.46, wherein a and X have the above-indicated meaning, are left to act upon the carbonic acid ester betaines according to formula (I).

12. A process according to claim 11 for the preparation of choline bitartrate.

13. A process according to claim 11 for the preparation of tricholine citrate, bis-choline tartrate, bis-choline hydrogen phosphate, choline dihydrogen phosphate, bis-choline hydrogen citrate, choline dihydrogen citrate, choline gluconate, choline salicylate, choline chloride, and choline ascorbate.

14. A process according to claim 11 for the preparation of choline carboxymethyl cellulose.

15. Carbonic acid ester betaine according to formula (I) 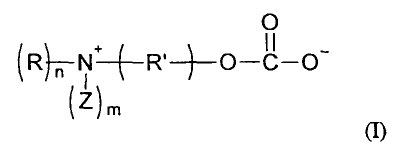 wherein R, R', Z, and m and n have the meaning given in claim 1, except for the compounds where n = 3.

## Revendications

1. Procédé pour la fabrication d'ester carbonique de bétaïne ayant la formule (I) 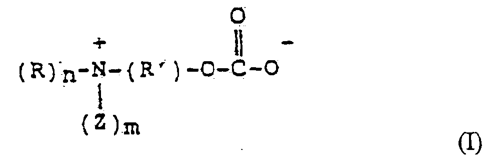 où R est un groupe alkyle ou différents groupes alkyles ayant 1 à 18 atomes de carbone, R' est un groupe alkylène ayant 2 à 12 atomes de carbone et Z est un groupe hydroxyalkyle ayant 1 à 12 atomes de carbone, m et n sont des nombres entiers, m est compris entre 0 et 3 et n est compris entre 3 et 0, et m+n=3, caractérisé en ce que l'on fait réagir une amine ayant la formule (II) 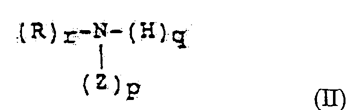 où R et Z sont définis comme précédemment, r, p et q sont des nombres entiers, r+p+q=3, r est compris entre 0 et 3, p est compris entre 3 et 0 et q est compris entre 0 et 2, avec un oxyde d'alkylène ayant 2 à 12 atomes de carbone en présence de dioxyde de carbone dans un agent dissolvant sensiblement inerte et en l'absence d'eau.

2. Procédé selon la revendication 1, caractérisé en ce que l'oxyde d'alkylène est l'oxyde d'éthylène.

3. Procédé selon l'une des revendications 1 et 2, caractérisé en ce que la réaction se déroule dans un agent dissolvant organique.

4. Procédé selon la revendication 3, caractérisé en ce que l'on utilise du méthanol comme agent dissolvant organique.

5. Procédé selon l'une des revendications 1 à 4, caractérisé en ce que l'on réalise la réaction avec un excès d'oxyde d'alkylène et avec un excès de dioxyde de carbone, par rapport à l'amine utilisée.

6. Procédé selon la revendication 5, caractérisé en ce que l'on utilise un excès de 1 à 200% molaire d'oxyde d'alkylène et 10 à 800% molaire de dioxyde de carbone.

7. Procédé selon l'une des revendications 1 à 6, caractérisé en ce que la réaction se déroule à une température comprise entre la température ambiante et 150°C.

8. Procédé selon l'une des revendications 1 à 7, caractérisé en ce que la réaction se déroule à une température comprise entre 40 et 80°C.

9. Procédé selon l'une des revendications 1 à 8, caractérisé en ce que la pression obtenue avec le dioxyde de carbone se trouve dans une gamme allant de 1 à 5 bars.

10. Procédé selon l'une des revendications 1 à 9, caractérisé en ce que la chambre de réaction est tout d'abord lavée avec un gaz inerte, de préférence de l'azote ou de l'argon, qui peut rester dans la chambre pendant la réaction.

11. Procédé pour la fabrication de sels ayant la formule (III) 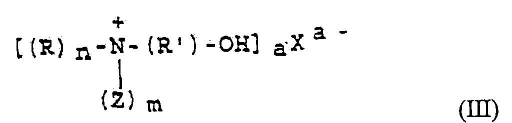 où R est un groupe alkyle ou différents groupes alkyles ayant 1 à 18 atomes de carbone, R' est un groupe alkylène ayant 2 à 12 atomes de carbone et Z est un groupe hydroxyalkyle ayant 1 à 12 atomes de carbone, m et n sont des nombres entiers, m est compris entre 0 et 3 et n est compris entre 3 et 0, m+n=3, X désigne l'anion d'un acide organique ou minéral et a est un nombre entier compris entre 1 et 3, caractérisé en ce que l'on fabrique tout d'abord de l'ester carbonique de bétaïne ayant la formule (I) 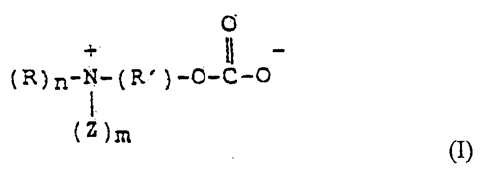 en faisant réagir une amine ayant la formule (II) 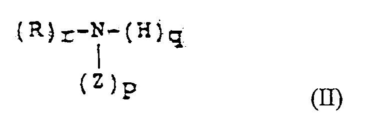 où R et Z sont définis comme précédemment, r, p et q sont des nombres entiers, r+p+q=3, r est compris entre 0 et 3, p est compris entre 3 et 0 et q est compris entre 0 et 2, avec un oxyde d'alkylène ayant 2 à 12 atomes de carbone en présence de dioxyde de carbone dans un agent dissolvant sensiblement inerte et en l'absence d'eau, après quoi on laisse réagir des acides HₐX^{a} ayant un pKₛ inférieur à 6,46, où a et X sont comme définis ci-dessus, sur l'ester carbonique de bétaïne ayant la formule (I).

12. Procédé selon la revendication 11 pour la fabrication de bitartrate de choline.

13. Procédé selon la revendication 11 pour la fabrication de citrate de tricholine, de tartrate de bis-choline, d'hydrogène-phosphate de bis-choline, de dihydrogène-phosphate de choline, d'hydrogène-citrate de bis-choline, de dihydrogène-citrate de choline, de gluconate de choline, de salicylate de choline, de chlorure de choline et d'ascorbate de choline.

14. Procédé selon la revendication 11 pour la fabrication de carboxyméthylcellulose de choline.

15. Ester carbonique de bétaïne ayant la formule (I) 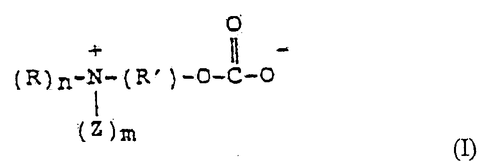 où R, R', Z, m et n sont comme définis dans la revendication 1, à l'exception des composés pour lesquels n=3.
